(19) Europäisches Patentamt
European Patent Office
Office européen des brevets

(11) Veröffentlichungsnummer: **0 037 373**
**A1**

(12) # EUROPÄISCHE PATENTANMELDUNG

(21) Anmeldenummer: **81810099.2**

(22) Anmeldetag: **16.03.81**

(51) Int. Cl.³: **C 07 C 51/09**
C 07 C 59/50, C 07 C 63/06
C 07 B 7/00, C 25 B 3/04
C 07 C 87/56, C 07 C 85/20
C 07 F 9/38, C 07 C 149/14
C 07 C 41/28, C 07 C 29/09

(30) Priorität: **21.03.80 CH 2240/80**

(43) Veröffentlichungstag der Anmeldung:
**07.10.81 Patentblatt 81/40**

(84) Benannte Vertragsstaaten:
**CH DE FR GB IT LI NL**

(71) Anmelder: **CIBA-GEIGY AG**
**Patentabteilung Postfach**
**CH-4002 Basel(CH)**

(72) Erfinder: **Scheffold, Rolf, Prof. Dr.**
**Multengutstrasse 31**
**CH-3074 Muri b. Bern(CH)**

(72) Erfinder: **Amble, Erik, Dr.**
**Jacob Aalls gate 1 B**
**Oslo 3(NO)**

(54) **Verfahren zur Eliminierung von Alkyl- und Alkenylgruppen.**

(57) Erfindungsgemäss kann eine an ein Sauerstoff- oder Schwefelatom gebundene Gruppe der Formel

$$-\underset{R^3}{\overset{R^2}{\underset{|}{\overset{|}{C}}}} - (CH{=}CH)_n - \underset{R^5}{\overset{R^4}{\underset{|}{\overset{|}{C}}}} - X \qquad (1)$$

worin R² und R³ unabhängig voneinander je ein Niederalkyl oder vorzugsweise Wasserstoff, R⁴ und R⁵ unabhängig voneinander je ein Niederalkyl, ein Aryl, Wasserstoff oder ein Halogen mit der Atomzahl von höchstens 35 und X eine elektrofuge Austrittsgruppe darstellen und n für 1 oder vorzugsweise 0 steht, unter überraschend milden, schonenden Bedingungen abgespalten werden, wenn man die Reduktion in Anwesenheit einer katalytischen Menge eines Uebergangsmetall-Komplexes einer Corrin- oder Porphin-Verbindung durchführt. Das Verfahren findet eine besondere Anwendung als eine selektive Methode zur Abspaltung von Gruppen der Formel I, insbesondere der 2-Chloräthylgruppe, welche zum vorübergehenden Schutz von verschiedenen sauerstoff- oder schwefelhaltigen funktionellen Gruppen, wie Carboxyl-, Phosphoryl-, Hydroxyl-, Carbonyl- und Aminogruppen, in eine organische Verbindung eingeführt wurden.

EP 0 037 373 A1

CIBA-GEIGY AG
Basel (Schweiz)

Case 4-12772/=

Verfahren zur Eliminierung von Alkyl- und Alkenylgruppen.

Gegenstand der vorliegenden Erfindung ist ein neuartiges Verfahren zur Abspaltung von β-substituierten Alkylgruppen vom Typ β-Halogenalkyl, die an einer sauerstoff- oder schwefelhaltigen funktionellen Gruppe gebunden sind, sowie von entsprechenden vinylogen δ-substituierten 2-Alkenylgruppen, durch die reduktive Auflösung der C-O- bzw. C-S-Bindung, und seine Verwendung zum vorübergehenden Schutze sauerstoff- und schwefelstoffhaltigen funktionellen Gruppen in polyfunktionellen organischen Verbindungen.

Es ist bekannt, dass die Festigkeit einer C-O-Bindung durch eine geeignete Substitution in der β-Stellung der entsprechenden Kohlenstoffkette beträchtlich gelockert werden kann, und dass die Abspaltbarkeit einer an einem Sauerstoffatom gebundenen Alkylgruppe durch die β-Substitution, z.B. mit einer elektrofugen Gruppe, wie einem Halogenatom, wesentlich vergrössert wird. Auf dieser allgemeinen Beobachtung basieren einige vorteilhafte Schutzgruppen für verschiedene geläufige funktionelle Gruppen organischer Verbindungen, und die Nützlichkeit des vorliegenden erfindungsgemässen Verfahrens lässt sich an ihrem Beispiel besonders anschaulich demonstrieren. Mehrere Gebiete der modernen organischen Chemie beschäftigen sich vorwiegend mit der Synthese polyfunktioneller Verbindungen, d.h. solcher, die im Molekül zwei oder mehrere Reaktionszentren gleicher oder verschiedener Art aufweisen, wie funktionelle Gruppen, mehrfache Bindungen, andere durch die Besonderheiten der Struktur aktivierte Atome u.s.w. Um eine chemische Umwandlung in die gewünschte Richtung selektiv zu lenken, ist es oft unumgänglich, nur das betreffende Zentrum (funktionelle Gruppe) als die alleinige verfügbare reaktive Stelle dem Angriff eines Reagens zugänglich zu belassen und die übrigen vorhandenen Reaktionszentren durch geeignete Schutzgruppen abzuschirmen und von der Teilnahme an der Umsetzung auszuschliessen. Dabei sollen geeignete Schutzgruppen zwei Voraussetzungen erfüllen: einerseits sollen sie eine möglichst hohe Stabilität gegenüber den üblichen Reaktionsmitteln und -bedingungen aufweisen, um die durchzuführenden Umwandlungen unversehrt durchzuhalten, andererseits müssen sie aber leicht abspaltbar sein und vorzugsweise eine möglichst hohe Empfinglichkeit gegenüber einem spezifischen Mittel besitzen, um sich einfach und ohne Beeinträchtigung des Syntheseproduktes, insbesondere anderer anwesender funktioneller Gruppen, entfernen zu lassen. Unter den funktionellen Gruppen, deren vorübergehender Schutz am häufigsten benötigt ist, stehen an erster Stelle Amino- und Carboxylgruppen, ferner auch andere saure sauerstoffhaltige Gruppen, z.B. solche, die als Zentralatom Phosphor haben, wie insbesondere organische Phosphate (Phosphorester), aber auch andere sauerstoff- oder schwefelhaltige Gruppen, wie Hydroxy- und Mercaptogruppen. Die Anwendung solcher Schutzgruppen erstreckt sich über den gesamten Bereich der synthetischen organischen Chemie; besonders häufig ist sie in der Chemie der Naturstoffe und deren Analogen, wie Steroide, Alkaloide, Hormone, z.B. Prostaglandine, und Kohlenhydrate und vor allem der Nukleinsäuren und Nukleotide, der Aminosäuren und Peptide, und der Antibiotica, wie der peptidartigen Antibiotica und ganz speziell der β-Lactam-Antibiotica der Penam- und Cepham-Reihe. Die eingangs erwähnten β-substituierten Alkylgruppen eignen sich als Schutzgruppen sehr gut und wurden, insbesondere die 2-Bromäthyl und 2,2,2-Trichloräthylgruppe, für verschiedene solche Aufgaben, z.B. zum Schutz von Amino-, Carboxyl- und Phosphatgruppen, mehrmals vorgeschlagen, vgl. z.B. die zusammenfassenden Abhandlungen in J.F.W. McOmie (Editor): "Protective Groups in Organic Chemistry" (Plenum Press, London, 1973), S. 57, 204 und 224-227; und in E. Wünsch (Herausgeber): "Synthese von Peptiden", Band XV/1 der Houben-Weyl "Methoden der organischen Chemie" (G. Thieme Verlag, Stuttgart, 1974), S. 99-104, 133 und 340-342. Als den grössten Vorteil dieser Gruppen betrachtet man ihre allgemeine Beständigkeit, insbesondere gegenüber basischen und vor allem sauren Bedingungen, diese Beständigkeit wirkt sich aber zugleich nachteilig aus, wenn man die Schutzgruppen mit klassischen Mitteln abzuspalten versucht. In einem vereinzelten Sonderfall erfolgte zwar die Abspaltung der β-Bromäthoxycarbonylgruppe (verwendet als Aminoschutzgruppe) durch die Austauschreaktion mit kostspieligem Silberperchlorat, aber die am häufigsten verwendete reduktive Abspaltung mit Zink erfordert im allgemeinen energische Reaktionsbedingungen, wie eine hohe Konzentration (etwa 90%) von Essigsäure und/oder höhere Reaktionstemperatur. Das Fehlen einer schonenden und selektiven, allgemein anwendbaren und dabei ökonomischen Abspaltungsmethode, insbesondere einer solchen für die besonders stabile 2-Chloräthylgruppe, ist offensichtlich der Grund dafür, dass eine breitere Anwendung dieser sonst sehr interessanten und nützlichen Kategorie der Schutzgruppen bis jetzt ausblieb.

Es wurde nun gefunden, dass durch das vorliegende erfindungsgemässe Verfahren auch sehr beständige Gruppen dieser Art unter überraschend milden, schonenenden Bedingungen abgespalten werden können. Die Erfindung besteht in einem Verfahren zur reduktiven Abspaltung einer an ein Sauerstoff- oder Schwefelatom gebundenen β-substituierten Alkylgruppe oder einer entsprechenden vinylogen δ-substituierten 2-Alkenylgruppe der allgemeinen Formel

$$-\underset{R^3}{\overset{R^2}{\underset{|}{\overset{|}{C}}}} - (CH=CH)_n - \underset{R^5}{\overset{R^4}{\underset{|}{\overset{|}{C}}}} - X \qquad (I)$$

worin $R^2$ und $R^3$ unabhängig voneinander je ein Niederalkyl oder vorzugsweise Wasserstoff, $R^4$ und $R^5$ unabhängig voneinander je ein Niederalkyl, ein Aryl, Wasserstoff oder ein Halogen mit der Atomzahl von höchstens 35 und X eine elektrofuge Austrittsgruppe darstellen und n für 1 oder vorzugsweise 0 steht, das dadurch gekennzeichnet ist, dass man die Reduktion in Anwesenheit einer katalytischen Menge eines Uebergangsmetall-Komplexes einer Corrin- oder Porphin-Verbindung durchführt.

Ein Niederalkyl ist ein solches mit 1-4 Kohlenstoffatomen und vorzugsweise mit einer geraden Kette, wie Aethyl, Propyl, Butyl und vor allem Methyl.

Ein Aryl ist ein Phenylrest, welcher durch 1 bis 3 Niederalkylreste, wie die oben genannten, insbesondere Methyl, substituiert sein kann; vorzugsweise ist es der unsubstituierte Phenylrest.

Die elektrofuge Austrittsgruppe X ist ein Anion-bildender Rest einer starken Säure, z.B. einer starken organischen Carbonsäure, wie der Trifluoracetoxy-Rest, einer organischen Sulfonsäure, wie der Mesyloxy- (Methansulfonyloxy-), Tosyloxy- (p-Toluolsulfonyloxy-), 4-Brombenzolsulfonyloxy- oder 4-Nitrobenzolsulfonyloxy-Rest, oder einer starken anorganischen Säure, wie der Schwefelsäure oder in erster Linie einer Halogenwasserstoffsäure vom Molekulargewicht zwischen 36 und 128, wie der Chlorid-, Bromid- oder Jodid-Rest (d.h. ein Chlor-, Brom- bzw. Jodatom).

Das Sauerstoff- bzw. Schwefelatom, an welches die abzuspaltende Gruppe der Formel I gebunden ist, ist das entständige Glied eines beliebigen Anion-bildenden organischen Rests, z.B. eines solchen von Carbonsäuren, Carbaminsäuren, Carbonaten, Phosphorhaltigen Säuren, wie Phosphaten, Pyrophosphaten, Phosphonaten und Phosphinaten, sowie Sulfaten und Alkoholen, bzw. von entsprechenden Schwefel-Analogen, wie insbesondere Thiocarbonsäuren, Dithiocarbonsäuren und Thiolen. Dementsprechend ist die Gruppe der Formel I auch als ein Bestandteil eines organischen Esters oder Aethers, bzw. Thioesters oder Thioäthers, zu betrachten. Polybasische Reste, wie z.B. Phosphate, können dabei auch zugleich zwei, meistenfalls gleiche, Gruppen der Formel I tragen.- Durch die Abspaltung der Gruppe der Formel I und Zutreten des Protons resultiert als Primärprodukt die entsprechende freie Säure oder Alkohol (bzw. Thiol).

Die zu reduzierende Verbindung (weiterhin auch als Substrat bezeichnet) kann einer beliebigen Kategorie der organischen Verbindungen, z.B. einer der oben genannten Naturstoffe, gehören und neben der behandelten Gruppierung noch weitere übliche funktionelle Gruppen oder sonstige Reaktionszentren aufweisen, z.B. freie, veresterte oder verätherte Hydroxylgruppen, freie und geschützte, wie ketalisierte, Oxogruppen, freie und veresterte Carboxylgruppen und ihre funktionelle Derivate, wie Nitrile, Amide und Hydrazide, Lactone, Lactame, insbesondere auch β-Lactame, Doppelbindungen, Dreifachbindungen, sowie Amino und substituierte Aminogruppen. Eine gewisse Vorsicht ist bei der Anwesenheit von leicht reduzierbaren funktionellen Gruppen, wie Nitro-, Azo- und Diazogruppen, sowie reaktiven Halogengruppen, z.B. einem Brom- oder insbesondere Jodatom,geboten; durch eine geeignete Wahl der Reduktionsbedingungen und des Reduktionsmittels ist oft auch in einem solchen Fall die Abspaltung selektiv durchführbar.

Uebergangsmetall-Komplexe von Corrin- und Porphin-Verbindungen, die man erfindungsgemäss als Katalysator der reduktiven Abspaltung einsetzt, leiten sich von solchen Uebergangsmetallen ab, die in mehr als einer Wertigkeitsstufe auftreten, wie Kupfer, Palladium, Rhodium und vor allem Kobalt. Bevorzugt als Katalysator sind Modellverbindungen zur Synthese von Vitamin $B_{12}$, z.B. Derivate, welche sich vom 2,2,3,3,7,7,8,8,12,12,13,13,17,17,18,18-Hexadecamethyl-10,20-diazahexahydroporphin-Kobalt(III)-Kation ableiten, und insbesondere Vitamine der $B_{12}$-Gruppe, darunter vor allem Cyanocobalamin, Aquocobalamin und Hydroxocobalamin, sowie ihre Derivate und Abbauprodukte, wie Cobyrinsäure und ihre Ester, Corphyrinsäure, Corphinsäure, Cobaminsäure und Cobamid. Die Menge des Katalysators beträgt im Normalfall etwa 0,1 bis etwa 0,001, vorzugsweise etwa 0,03 bis etwa 0,003 Moläräquivalent, bezogen auf die zu reduzierende Komponente, bei einer günstigen Versuchsanordnung, z.B. bei einer optimalisierten Elektroreduktion, kann man aber die unterste Grenze noch weit unterschreiten.

Die Reduktion wird in an sich bekannter Weise durch konventionelle Methoden in Anwesenheit einer Protonenquelle durchgeführt. Als diese können beliebige anorganische und organische Säuren sowie gebräuchliche protische Lösungsmittel dienen; Säuren werden mit Vorteil in einer gepufferten Form oder als Salze eingesetzt, wobei der pH-Wert vorzugsweise zwischen etwa 4,0 bis 9,5, insbesondere zwischen etwa 5,5 bis 7,5 und vor allem in der Nähe des Neutralpunktes liegt. Bei der spezifischen Auswahl der Protonenquelle werden die üblichen Kriterien berücksichtigt, wie die Natur der zu reduzierenden Verbindung (Substrats) und die Besonderheiten der angewendeten Reduktionsmethode, um optimale Entfernung der Nebenprodukte zu ermöglichen.

Der Natur des Substrats nach richtet sich ebenfalls die engere Auswahl der Reduktionsmethode und des Reduktionsmittels, wobei die Verträglichkeit mit übrigen Reaktionszentren des Substrats im besonderen in Erwägung gezogen werden muss. Als eine der schonendsten Reduktionsmethoden empfiehlt sich die kathodische Elektroreduktion unter konventionellen Bedingungen, wie an der Quecksilber- oder einer anderen für die Elektroreduktion allgemein verwendbaren Kathode, wie Kohle-Kathode, in Anwesenheit von üblichen Hilfselektrolyten, wie Ammonium- und/oder Alkalimetallsalzen, insbesondere Lithiumsalzen, starker anorganischer Säuren, wie insbesondere Halogeniden und Perchloraten, vorzugsweise solchen, die sich durch eine gute Löslichkeit im verwendeten wässrig-organischen Milieu auszeichnen. Als Lösungsmittel dienen, neben Wasser, übliche mit Wasser mischbare organische Lösungsmittel, z.B. Niederalkanole, wie Methanol, Aethanol und Isopropylalkohol, oder Dimethylformamid und ähnliche niederaliphatische Amide, ferner auch Nitrile, wie Acetonitril, Aether, wie Diäthyläther, 1,2-Dimethoxy- und 1,2-Diäthoxy-äthan und cyclische Aether vom Typ Tetrahydrofuran, Ketone, wie Aceton, Carbonate, wie Dimethyl- und Diäthylcarbonat, sowie Sulfoxide, wie insbesondere Dimethylsulfoxid. Die Temperatur der Elektroreduktion kann in einem breiten Bereich variiert werden, d.h. von etwa -20°C bis zur Siedetemperatur des Reaktionsgemisches; vorzugsweise liegt sie unterhalb

oder bei der Raumtemperatur. - Als eine weitere geeignete Variante der reduktiven Abspaltung kommt die Reduktion mit einem Metall, vor allem mit Zink oder auch mit Magnesium, in Betracht. Dabei kann man unter Umständen die üblichen energischen Bedingungen, z.B. etwa 90%-ige wässrige Essigsäure als Milieu und/oder erhöhte Temperatur bis zum Siedepunkt des Gemisches bei entsprechend verkürzter Reaktionszeit anwenden, vorzugsweise arbeitet man aber unter möglichst schonenden Bedingungen, z.B. im oben erwähnten bevorzugten pH-Bereich in der Nähe des Neutralpunktes und bei Temperaturen von etwa 0° bis etwa 40°C, vorzugsweise etwa 10-25°C; üblicherweise arbeitet man mit einem etwa zehnfachen molaren Ueberschuss am Metall, vorzugsweise Zink in Form von Zinkstaub, den man mit Vorteil noch unmittelbar vor der Reaktion in der konventionellen Weise, z.B. durch Verrühren mit verdünnter Salzsäure und sorgfältiges Neutralwaschen, aktiviert. Zum Erzielen der gewünschten Pufferwirkung können mit Vorteil Ammoniumsalze von Halogenwasserstoffsäuren dienen, insbesondere von denjenigen, die in der jeweiligen abzuspaltenden Gruppe der Formel I vorkommen. Die Reduktion erfolgt in den oben erwähnten organischen Lösungsmitteln oder deren Gemischen; protische Lösungsmittel sind bevorzugt. Mit Rücksicht auf die jeweilige Natur der zu reduzierenden Verbindung (Substrats) sind auch andere herkömmliche Varianten des Reduktionsverfahrens anwendbar: als Beispiel sei erwähnt die Reduktion mit Metall-Kationen in tiefen Wertigkeitsstufen, z.B. mit Chrom(II)-Salzen, wie Chrom(II)-chlorid oder -acetat, ferner auch die Reduktion mit komplexen Metallhydriden, z.B. mit Natriumborohydrid und verwandten Niederalkoxy-natriumborohydriden und Niederalkoxy-lithiumborohydriden, sowie die katalytische Hydrierung, z.B. an einem Palladium-Katalysator.

Obwohl die erfindungsgemässe katalytische Abspaltung, wie oben gezeigt wurde, im ganzen Bereich der organischen Chemie ihre Geltung findet, ist ihr besonders vorteilhaftes Anwendungsgebiet im vorübergehenden Schutz von Sauerstoff- oder Schwefelhaltigen funktionellen

Gruppen zu suchen. Diese besondere Ausführung der vorliegenden Erfindung ist dadurch gekennzeichnet, dass man zum vorübergehenden Schutz einer Anion-bildenden sauerstoff- oder schwefelhaltigen funktionellen Gruppe die oben definierte Gruppe der Formel I an das entsprechende Sauerstoff- bzw. Schwefelatom einführt und in einem gewünschten späteren Synthesestadium in der oben definierten Weise durch die erfindungsgemäss katalysierte Reduktion abspaltet.

Erfindungsgemäss kann man in erster Linie eine Carboxylgruppe in Form eines Alkyl- bzw. 2-Alkenylesters, dem die Gruppe der Formel I zugrunde liegt, schützen. Die Schutzgruppe der Formel I kann man in an sich bekannter Weise, in der Regel durch Veresterung mit einem entsprechenden β-substituierten Alkanol (bzw. dem entsprechenden Vinylogen) gemäss dem bekannten Stand der Technik einführen. Diese Schutzmethode ist besonders vorteilhaft, wenn zugleich die herkömmlichen acidolytisch und reduktiv abspaltbaren Amino-, Carboxyl- und Hydroxyl-Schutzgruppen im Substrat vorhanden sind. Als Substrat sind besonders Verbindungen der β-Lactamgruppe, insbesondere Antibiotica von Penam- und Cepham-Ringsystem und ihre synthetischen Vorstufen und Analoge, sowie Aminosäuren und Peptide geeignet.

Durch die Gruppe der Formel I, kann man erfindungsgemäss auch die Phosphatgruppe in Form eines entsprechenden Mono- oder Diesters schützen, wie es insbesondere durch die Teilformel

```
      O                      O
      ||                     ||
  -O-P-O-(I)      bzw.   -O-P-O-(I)
      |                      |
    - O                      O - (I)
```

dargestellt wird, worin (I) den oben definierten Rest der Formel I bedeutet, wobei der ganze Phosphatrest mit mindestens einer seiner freien Valenzen an ein C-Atom eines beliebigen organischen Rests, insbesondere eines Nucleosids, durch die Esterbindung gebunden wird. In analoger Weise kann auch ein Pyrophosphat und andere saure Reste mit

zentralem Phosphoratom geschützt werden. Die Einführung der Schutzgruppe erfolgt in der bekannten üblichen Weise, z.B. durch die Veresterung des zu schützenden Monoesters (z.B. eines Nucleosidphosphats) oder Diesters (z.B. eines Dinucleosidphosphats) mit einem entsprechenden β-substituierten Alkanol (bzw. einem Vinylogen davon) oder aber durch die Kondensation eines Mono- oder Diphosphats des β-substituierten Alkanols (oder 2-Alkenols), vorzugsweise in einer aktivierten Form, z.B. als ein gemischtes Anhydrid, mit der zu schützenden, aber noch nicht phosphorylierten Hydroxyverbindung (z.B. einem Nucleosid).

Durch die Gruppe der Formel I kann erfindungsgemäss auch eine Hydroxyl- oder Thiolgruppe geschützt werden. Dazu wird entweder das zu schützende Sauerstoff- bzw. Schwefelatom durch diese Schutzgruppe direkt veräthert, oder aber durch einen entsprechenden Monocarbonat-Rest der Teilformel -C(=O)-O-(I), worin (I) die oben definierte Gruppe der Formel I bedeutet, verestert. Der zu schützende Alkohol (bzw. Thiol) kann einer beliebigen Kategorie organischer Verbindungen, insbesondere der oben erwähnten Naturstoffe, wie Zucker, Nucleosiden oder Peptiden, angehören. Die Einführung der Schutzgruppe kann in der konventionellen Weise erfolgen, als eine vorteilhafte Methode zur Einführung des Monocarbonat-Rests ist die Behandlung des zu schützenden Alkohols (Thiols) mit dem entsprechenden Chloroformiat der Formel Cl-CO-O-(I) besonders zu erwähnen. Falls der Schutz über den Monocarbonat-Rest erfolgt, verläuft die erfindungsgemässe reduktive Abspaltung so, dass der nach der Abspaltung der Gruppe I primär entstandene unstabile Hälbester gleich spontan decarboxyliert und die Hydroxylgruppe freisetzt.

In einem Sonderfall des unmittelbar vorangehend beschriebenen erfindungsgemässen Schutzes von Hydroxylgruppen können zwei solche durch die Gruppe der Formel I geschützte Hydroxylgruppen sich auf einem und demselben Kohlenstoffatom befinden. So bilden sie dann eine Acetal- oder Ketalgruppe der Formel

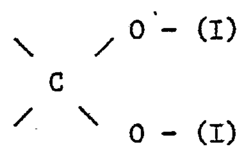

worin (I) die oben definierte Gruppe der Formel I darstellt und eine der freien Valenzen mit einem Kohlenstoffatom eines beliebigen organischen Rests und die andere mit einem Wasserstoffatom oder einem Kohlenstoffatom eines beliebigen organischen Rests gebunden ist, wobei die beiden organischen Reste gleich oder verschieden sein oder zusammen einen zweiwertigen organischen Rest bilden können. In dieser Form findet die Gruppe I erfindungsgemäss die Anwendung zum Schutze von Oxogruppen (Carbonylgruppen)sowohl des aldehydischen wie ketonischen Charakters. Die Einführung der Schutzgruppe erfolgt in an sich bekannter Weise durch konventionelle allgemeine Methoden der Acetalisierung bzw. Ketalisierung, z.B. durch Behandeln der zu schützenden Oxoverbindung mit einem entsprechenden β-substituierten Alkanol (bzw. seinem Vinylogen) oder einem reaktionsfähigen Derivat davon, wie einem Acetonid oder Butanon-Ketal, unter Säure-Katalyse. Die erfindungsgemässe reduktive Abspaltung erfolgt vorzugsweise in einem praktisch neutralen oder schwach basischen Milieu. Als die beiden Schutzgruppen der Formel I werden bei dieser Variante vorzugsweise die 2-Bromäthyl- und insbesondere die 2-Chloräthylgruppe verwendet, oder, mit einem ganz speziellen Vorteil, die beiden Gruppen der Formel I werden in einer einzigen, der Gruppe I analogen, zweiwertigen Schutzgruppe, wie der 2,2-Dibrompropa-1,3-diyl-, 2-Chlorpropa-1,3-diyl- oder 3-Chlorpropa-1,2-diyl-Gruppe der Formeln

$\underset{|}{C}H_2-CBr_2-\underset{|}{C}H_2$ , $\underset{|}{C}H_2-CHCl-\underset{|}{C}H_2$ bzw. $\underset{|}{C}H_2-\underset{|}{C}H-CH_2Cl$ , oder

anderen solchen Gruppen , kombiniert, welche dann zusammen mit der so geschützten Carbonylgruppe einen 1,3-Dioxolan- bzw. 1,3-Dioxanring bildet. Die letztgenannte spezielle Variante bietet noch den zusätzlichen Vorteil, dass cyclische Acetale und Ketale aus den zu schützenden Oxoverbindungen und entsprechenden Diolen (z.B. dem 3-Chlorpropan-1,2-diol oder 2-Chlorpropan-1,3-triol) mit besonderer Leichtigkeit entstehen. Analog, wie oben erwähnt wurde, können diese Diole in Form eines reaktionsfähigen Derivats, z.B. eines Ketals mit einem leicht flüchtigen Keton, wie Aceton oder Butan-2-on, eingesetzt werden. Vornehmlich wird das erfindungsgemässe Verfahren zum Schutze von Oxogruppen in Steroidverbindungen, insbesondere von Oxogruppen in 3-, 17-, 18-, 19- und/oder 20-Stellung, verwendet. Da die konventionellen Schutzmethoden zur Entfernung der Schutzgruppe säurekatalysierte Hydrolyse, oft unter energischen Bedingungen, erfordern, kommt hier der spezielle Vorteil des erfindungsgemässen Verfahrens, nämlich die Abspaltung unter praktisch neutralen Bedingungen, besonders deutlich zur Geltung.

Die Gruppe der Formel I kann man erfindungsgemäss auch mit besonderem Vorteil zum Schutze von Aminogruppen anwenden, indem man die zu schützende Aminogruppe, d.h. eine primäre oder sekundäre Aminogruppe in einen entsprechenden Carbaminsäureester umwandelt, der als die Estergruppe die oben definierte Gruppe der Formel I hat, und die Schutzgruppe in einem späteren Stadium durch das erfindungsgemässe reduktive Verfahren abspaltet. Die geschützte Aminogruppe ist durch die Formel $>N-C(=O)-O-(I)$ charakterisiert, worin (I) die oben definierte Gruppe I darstellt und worin beide freien Valenzen je an ein Kohlenstoffatom eines beliebigen zweiwertigen organischen Rests oder zweier beliebiger selbstständiger, gleicher oder verschiedener, einwertiger organischer Reste, oder vorzugsweise eine der Valenzen an ein Wasserstoffatom gebunden sind. Die Entfernung der ganzen Schutzgruppe verläuft dann so, dass durch die erfindungsgemässe

reduktive Abspaltung der Gruppe I die unstabile Carbaminsäure primär entsteht, welche dann unter Freisetzung der Aminogruppe spontan oder bei Aufarbeitung, z.B. bei einem vorübergehenden Ansäuern, decarboxyliert. Die Einführung der entsprechenden Schutzgruppe, d.h. der β-substituierten Alkoxycarbonyl- bzw. der vinylogen 2-Alkenyloxycarbonylgruppe der Formel -C(=O)-O-(I), worin (I) die oben definierte Gruppe I darstellt, kann in der herkömmlichen bekannten Weise erfolgen, z.B. durch die Behandlung der zu schützenden Aminoverbindungen mit dem entsprechenden Chloroformiat der Formel Cl-C(=O)-O-(I). Als Substrat können beliebige organische Verbindungen eingesetzt werden, von besonderem Vorteil ist jedoch dieser Schutz bei Aminosäuren und Peptiden, sowie bei Verbindungen des β-Lactam-Typs.

Die erfindungsgemäss angewendete Schutzgruppe kann in einer und derselben Verbindung auch zum Schutze mehrerer, gleicher oder insbesondere verschiedenartiger, funktioneller Gruppen eingesetzt werden. Dies ist insbesondere bei der Peptid-Synthese vorteilhaft, da man dadurch in einer einzigen Endstufe sämtliche gebliebene Schutzgruppen auf einmal entfernen kann. Eine ähnliche Arbeitsweise kann auch bei den Verbindungen des β-Lactam-Typs von Vorteil sein. Unter Berücksichtigung der bekannten spezifischen chemischen Eigenschaften jeder einzelnen dieser Gruppen kann man sie, dank dem erfindungsgemässen Verfahren, in zweckmässigen Kombinationen untereinander und mit anderen herkömmlichen Schutzgruppen zum selektiven Schutze der oben geschilderten sauerstoff- und schwefelhaltigen Anion-bildenden funktionellen Gruppen auf dem ganzen Gebiet der organischen Chemie einsetzen.

Unter den erfindungsgemäss abspaltbaren Gruppen der Formel I sind für die Anwendung als Schutzgruppe die folgenden besonders geeignet und bevorzugt: die 4-Chlorcrotyl- und 4-Bromcrotyl-, die 2-Bromäthyl und 2,2-Dichloräthyl, und vor allem die 2,2,2-Trichloräthyl- und 2-Chloräthylgruppe.

Durch die nachfolgenden Beispiele wird einerseits die Durchführbarkeit des erfindungsgemässen Verfahrens unter Bedingungen demonstriert, bei welchen die konventionelle Reduktion versagt, andererseits einige Varianten zur Illustrierung der breiten Anwendbarkeit des Verfahrens vorgeführt, ohne dadurch den Umfang der Erfindung in irgend einer Hinsicht zu beschränken. Die Temperaturen sind in Grad Celsius angegeben.

Beispiel 1:

a) 78 mg (0,10 mMol) Katalysator [1-Hydroxy-2,2,3,3,7,7,8,8,12,12,13,13,17,17,18,18-hexadecamethyl-10,20-diazaoctahydroporphinato]-dibromo-cobalt(III) und 2,0 g (37,4 mMol) Ammoniumchlorid in 30 ml Aethanol/Wasser (1:1) werden unter Argon mit 0,5 g (7,6 mMol) frisch aktiviertem Zinkstaub (aktiviert durch Waschen mit 0,1N HCl, Spülen mit Wasser, dann Aethanol und Aether und Trocknen) und 0,46 g (2,5 mMol) Benzoesäure-β-chloräthylester versetzt und bei Raumtemperatur gerührt. Nach 15 Std. wird mit 50 ml Methylenchlorid und 50 ml Wasser versetzt, vom Zink abfiltriert und mit KOH der pH auf 11 gestellt. Nach 4maligem Extrahieren mit 50 ml Methylenchlorid wird mit verdünnter Schwefelsäure angesäuert (pH = 3) und mit Aether (3 x 100 ml) extrahiert. Die Aetherlösung wird 2mal mit 50 ml Wasser gewaschen, mit Magnesiumsulfat getrocknet und eingeengt. Es resultieren 0,16 g leicht gelbliche Kristalle von Benzoesäure, Smp. 122 - 123°. Ausbeute: 53 %.

b) Ohne Katalysator: In analoger Weise wie unter a), aber ohne Katalysator, wurden 0,46 g (2,5 mMol) Benzoesäure-β-chloräthylester, 0,5 g (7,6 mMol) frisch aktiviertes Zink (siehe Beispiel 1a) und 2,0 g (37,4 mMol) Ammoniumchlorid mit 30 ml Aethanol/Wasser (1:1) versetzt und bei Zimmertemperatur während 16 Stunden gerührt. Dann wurde mit Wasser (50 ml) und Aether (50 ml) verdünnt, vom Zink abfiltriert, mit 25% Salzsäure auf pH 3 angesäuert und 3mal mit Aether extrahiert. Die Aetherphase wurde 2mal mit 50 ml Wasser gewaschen, mit Magnesiumsulfat getrocknet und eingedampft. Nach Abdampfen des Aethers verblieben 0,43 g einer farblosen Flüssigkeit, die auf Grund ihres $^1$H-NMR-Spektrums und $R_f$-Wertes als der regenerierte Ausgangsstoff identifiziert wurde; Ausbeute: 93%.

Beispiel 2:

25 mg (0,018 mMol) Aquocobalamin und 11,0 g (206 mMol) Ammoniumchlorid in 50 ml Aethanol/Wasser (1:1) werden unter Argon mit 11,8 g (181 mMol) Zinkstaub versetzt. Nach 15 Min. Rühren bei Raumtemperatur wird 3,34 g (18,1 mMol) Benzoesäure-β-chloräthylester zugegeben und zwei Tage lang bei Raumtemperatur gerührt. Nach der üblichen Aufarbeitung gemäss Beispiel 1 a) erhält man 1,39 g (63% d.Th.) Benzoesäure vom Smp. 121 - 122°.

Beispiel 3:

a) 100 mg (0,07 mMol) Katalysator (Aquocobalamin) und 0,20 g (2,0 mMol) Ammoniumbromid werden unter Erwärmung in 30 ml Aethanol/Wasser (1:1) gelöst und unter Argon mit 1,60 g (25 mMol) frisch aktiviertem Zinkstaub (siehe Beispiel 1) versetzt, worauf sich die Suspension rasch blaugrün färbt, und 0,57 g (2,5 mMol) Benzoesäure-β-bromäthylester dazugegeben, wobei ein momentaner Farbumschlag nach rot erfolgt. Nach 3 Std. Rühren bei Zimmertemperatur unter Argon wird die wiederum blaugrüne Suspension mit 100 ml Wasser und 100 ml Aether verdünnt,

vom Zink abfiltriert, mit 25% Salzsäure auf pH 3 angesäuert und mit 100 ml Aether extrahiert. Die Aetherlösung wird 2mal mit 50 ml Wasser gewaschen, über Magnesiumsulfat getrocknet und eingedampft. Man erhält 0,23 g eines farblosen, festen Rückstandes der Benzoesäure, Smp. 122°, in der Ausbeute von 77%.

b) <u>Ohne Katalysator:</u> In analoger Weise wie unter a) wurde 0,57 g (2,5 mMol) Benzoesäure-β-bromäthylester zu einer Suspension von 1,60 g (24,5 mMol) Zinkstaub (aktiviert, wie im Beispiel 1 angegeben) und 0,20 g (2,0 mMol) Ammoniumbromid in 30 ml Aethanol/Wasser (1:1) gegeben und während 3 Std. unter Argon bei Raumtemperatur gerührt. Dann wurde mit Wasser (50 ml) und Aether (50 ml) verdünnt, vom Zink abfiltriert, mit 25% Salzsäure auf pH 3 angesäuert und 3mal mit Aether extrahiert. Die Aetherphase wurde 2mal mit 50 ml Wasser gewaschen, mit Magnesiumsulfat getrocknet und eingedampft. Nach Abdampfen des Aethers verblieben 0,37 g einer farblosen Flüssigkeit, die auf Grund ihres $^{1}$H-NMR-Spektrums aus dem regenerierten Ausgangsstoff bestand; Ausbeute: 65%.

<u>Beispiel 4:</u>

83 mg (0,107 mMol) Katalysator [1-Hydroxy-2,2,3,3,7,7,8,8,12,12,13,13,17,17,18,18-hexadecamethyl-10,20-diazaoctahydroporphinato]-dibromo-cobalt(III), 2,5 g (25 mMol) Ammoniumbromid und 1,6 g (25 mMol) frisch aktivierter Zinkstaub (siehe Beispiel 1) in 40 ml Aethanol/Wasser (1:1) werden unter Argon bei Zimmertemperatur mit 0,57 g (2,5 mMol) Benzoesäure-β-bromäthylester versetzt und während 4 Tagen gerührt. Nachdem wie im Beispiel 3 aufgearbeitet wird, erhält man 0,30 g (100%) Benzoesäure, Smp. 122°.

Beispiel 5:

30 mg (0,022 mMol) Cyanocobalamin (als Katalysator) und 0,76 g (7,6 mMol) Ammoniumbromid in 30 ml Aethanol/Wasser (1:1) werden mit 0,50 g (7,6 mMol) aktiviertem Zinkstaub (siehe Beispiel 1) versetzt, worauf die Farbe allmählich über braunrot nach blaugrün umschlägt. Nach Zugabe von 0,186 g (0,81 mMol Benzoesäure-β-bromäthylester (momentaner Farbumschlag nach rot) und weiteren 1,72 g (17,6 mMol) Ammoniumbromid wird während zweier Tage unter Argon bei Zimmertemperatur gerührt, dann wie im Beispiel 3 aufgearbeitet. Man erhält 0,10 g (100%) Benzoesäure, Smp. 121-122°.

Beispiel 6:

Zu 50 mg (0,036 mMol) Aquocobalamin und 3,0 g (56,1 mMol) Ammoniumchlorid in 30 ml Aethanol/Wasser (1:1) wird unter Argon 0,5 g (7,6 mMol) Zinkstaub (aktiviert wie im Beispiel 1) und 0,53 g (2,5 mMol) N-Tolyl-carbamidsäure-2-chloräthylester gegeben und während 17 Std. bei Zimmertemperatur unter Argon gerührt. Das Gemisch wird mit 50 ml Wasser und 50 ml Methylenchlorid verdünnt, vom Zink abfiltriert und mit ca. 10-proz. KOH auf pH 11 gestellt. Nach 6maligem Extrahieren mit 50 ml Methylenchlorid, Waschen mit 2mal 50 ml Wasser, Trocknen mit Magnesiumsulfat und Eindampfen der Lösung erhält man 0,26 g eines leicht gelblichen Oels, das beim Abkühlen kristallisiert und auf Grund des $^1$H-NMR-Spektrums und $R_f$-Wertes als praktisch reines Toluidin ist; Ausbeute: 96%.

Beispiel 7:

a) 50 mg (0,037 mMol( Cyanocobalamin, 3,0 g (56,1 mMol) Ammoniumchlorid, 0,5 g (7,6 mMol) Zinkstaub und 0,71 g (2,5 mMol) N-Tolyl-carbamidsäure-2,2,2-trichloräthylester in 30 ml Aethanol/Wasser (1:1) werden unter Argon während 63 Std. bei Zimmertemperatur gerührt. Nach Aufarbeitung wie im Beispiel 6 erhält man 0,23 g leicht gelbliche Kristalle, die nach $^1$H-NMR-Spektrum und Dünnschicht-

chromatographie reines Toluidin darstellen; Ausbeute 85% d.Th.

b) Ohne Katalysator: In gleicher Weise wie unter a), jedoch ohne Katalysator, wurden 0,71 g (2,5 mMol) N-Tolyl-carbamidsäure-2,2,2-trichloräthylester, 3,0 g (56,1 mMol) Ammoniumchlorid und 0,5 g (7,6 mMol) Zinkstaub in 30 ml Aethanol/Wasser (1:1) bei Zimmertemperatur während 63 Std. gerührt. Die Aufarbeitung erfolgte wie im Beispiel 6. Nach Abdampfen des Lösungsmittels verblieben 0,31 g eines gelben Oels, das auf Grund des $^{1}H$-NMR-Spektrums und der Dünnschichtchromatographie aus Toluidin, dem Ausgangsstoff und einer weiteren, nicht identifizierten Verbindung besteht.

Beispiel 8:

0,57 g (2,2 mMol) Mandelsäure-β-bromäthylester wird in 22 ml Elektrolyt (0,1N $LiClO_4$ und 0,13N $NH_4ClO_4$ in Dimethylformamid) gelöst und in Gegenwart von 0,110 g (0,079 mMol) Aquocobalamin bei konstantem Potential von -1,99 V (gegen $Ag/Ag^+$) an der gerührten Quecksilberelektrode im Kathodenraum einer Halbzelle bei 10° elektrolysiert. Nach 6 Std. wird die Lösung mit 50 ml Wasser verdünnt, mit 25% Salzsäure auf pH = 3 angesäuert und 5 mal mit 50 ml Aether extrahiert. Nach zweimaligem Waschen mit 50 ml Wasser und Trocknen mit Magnesiumsulfat wird der Aether abgedampft. Der kristallisierende Rückstand (0,27 g) enthält gemäss $^{1}H$-NMR-Spektrum und Dünnschichtchromatogramm Mandelsäure neben etwas Dimethylformamid. Nach 2maligem Umkristallisieren wird Mandelsäure vom Smp. 119-120° in einer Ausbeute von ca. 45% isoliert.

Beispiel 9:

0,46 g (2,5 mMol) Benzoesäure-β-chloräthylester in 22 ml Elektrolyt (0,1N NaCl und 0,12N $NH_4Cl$ in Aethanol/Wasser (4:1) werden in Gegenwart von 100 mg (0,07 mMol) Aquocobalamin bei konstantem Potential von -1,99 V bei 10°C unter den gleichen Bedingungen

wie im Beispiel 8 elektrolysiert. Nach 30 Std. wird mit 50 ml Wasser verdünnt, mit 25% Salzsäure auf pH = 1 angesäuert und mit 3mal 50 ml Aether extrahiert. Die Aetherlösung wird 2mal mit je 50 ml Wasser gewaschen, über Magnesiumsulfat getrocknet und eingedampft. Der farblose, feste Rückstand ist Benzoesäure vom Smp. 119-120°; Ausbeute 0,30 g (98%).

Beispiel 10:

0,57 g (2,5 mMol) Benzoesäure-β-bromäthylester in 23 ml Elektrolyt (0,1N $LiClO_4$ und 0,13N $NHClO_4$ in Dimethylformamid) werden in Gegenwart von 100 mg (0,07 mMol) Aquocobalamin bei -1,99 V wie im Beispiel 8 elektrolysiert. Nach 6 Std. wird mit 200 ml Wasser verdünnt, mit 25% Salzsäure auf pH = 2 angesäuert und 4mal mit 100 ml Aether extrahiert. Die Aetherlösung wird mit 2mal 50 ml Wasser gewaschen, über Magnesiumsulfat getrocknet und eingedampft, womit 0,25 g (83%) Benzoesäure, Smp. 117-118°, resultiert.

Beispiel 11:

0,57 g (2,5 mMol) Benzoesäure-β-bromäthylester wird in 23 ml Elektrolyt (0,1N $LiClO_4$ und 0,13N $NH_4ClO_4$ in Methanol) gelöst und in Gegenwart von 100 mg (0,07 mMol) Aquocobalamin bei -1,99 V und 10°C wie im Beispiel 8 elektrolysiert. Nach 3 Std. wird wie im Beispiel 9 aufgearbeitet. Nach dem Verdampfen des Lösungsmittel resultiert 0,23 g (75%) verunreinigte Benzoesäure vom Smp. 109-110°; nach Umkristallisation aus Wasser 122-123°.

Beispiel 12:

0,57 g (2,5 mMol) Benzoesäure-β-bromäthylester wird in 25 ml Elektrolyt (0,1N $LiClO_4$ und 0,16N $NH_4ClO_4$ in 94% Aethanol) gelöst und in Gegenwart von 74 mg (0,095 mMol) Katalysator [1-Hydroxy-2,2,3,3,7,7,8,8,12,12,13,13,17,17,18,18-hexadecamethyl-10,20-diazaoctahydroporphinato]-dibromo-cobalt(III) bei -1,70 V und 10°C wie im Beispiel 8

elektrolysiert. Nach 13 Std. wird mit je 50 ml Methylenchlorid und Wasser verdünnt, der pH auf 12 mit KOH gestellt und mit 3mal 50 ml Methylenchlorid extrahiert. Dann wird mit 25% Salzsäure auf pH = 2 angesäuert und 3mal mit Methylenchlorid extrahiert, mit Wasser gewaschen, mit Magnesiumsulfat getrocknet und eingedampft. Die Ausbeute an leicht gelblicher Benzoesäure beträgt 0,27 g (90% d.Th.), Smp. 122 - 123°.

Beispiel 13:

0,79 g (1,73 mMol) Pen-V-säure-β-bromäthylester in 25 ml Elektrolyt (0,1N $LiClO_4$ und 0,13N $NH_4ClO_4$ in Aethanol/Wasser (4:1)) werden in Gegenwart von 95 mg (0,07 mMol) Aquocobalamin bei 1°C und -1,99 V wie im Beispiel 8 elektrolysiert. Nach 17 Std. wird mit je 50 ml Wasser und Methylenchlorid verdünnt, 6mal mit 50 ml Methylenchlorid extrahiert und mit 2mal 50 ml Wasser gewaschen. Nach Trocknen mit Magnesiumsulfat und Eindampfen erhält man 0,57 g eines leicht bräunlichen, zähflüssigen Rückstandes, der auf Grund des $^1$H-NMR-Spektrums und der Dünnschichtchromatographie als Pen-V-säure identifiziert wird; Ausbeute praktisch quantitativ.

(Unter der Bezeichnung Pen-V-säure ist die 6-Phenoxyacetamidopenicillansäure, d.h. 3,3-Dimethyl-7-oxo-6-phenoxyacetylamino-4-thia-1-azabicyclo[3,2,0]heptan-2-carbonsäure, zu verstehen.)

Beispiel 14:

0,30 g (0,68 mMol) Cephemsäure-β-bromäthylester in 25 ml Elektrolyt (0,1N $LiClO_4$ und 0,14N $NH_4ClO_4$ in 94% Aethanol) werden in Gegenwart von 30 mg (0,022 mMol) Aquocobalamin bei 20°C und -1,99 V wie im Beispiel 8 elektrolysiert. Nach 6 Std. wird mit je 100 ml Wasser und Methylenchlorid verdünnt, 4mal mit 100 ml Methylenchlorid extrahiert und mit 2mal 60 ml Wasser gewaschen. Nach Trocknen mit Magnesiumsulfat und Eindampfen erhält man 0,15 g farblose Kristalle der Cephemsäure, gemäss dem IR-Spektrum, $^1$H-NMR-

Spektrum und der Dünnschichtchromatographie mit einer authentischen Probe identisch; Ausbeute 66% d.Th.

(Unter der Bezeichnung Cephemsäure ist 7-Phenylacetamido-3-methyl-$\Delta^3$-cephem-4-carbonsäure, d.h. 3-Methyl-8-oxo-7-phenylacetylamino-5-thia-1-azabicyclo[4,2,0]oct-2-en-2-carbonsäure, zu verstehen.)

Patentansprüche

1. Verfahren zur Abspaltung einer β-substituierten Alkylgruppe oder einer entsprechenden vinylogen δ-substituierten 2-Alkenylgruppe, welche beide durch die Formel

$$-\underset{R^3}{\overset{R^2}{\underset{|}{\overset{|}{C}}}}-(CH{=}CH)_n-\underset{R^5}{\overset{R^4}{\underset{|}{\overset{|}{C}}}}-X \qquad (I),$$

worin $R^2$ und $R^3$ unabhängig voneinander je ein Niederalkyl oder Wasserstoff, $R^4$ und $R^5$ unabhängig voneinander je ein Niederalkyl, ein Aryl, Wasserstoff oder ein Halogen mit der Atomzahl von höchstens 35 und X eine elektrofuge Austrittsgruppe darstellen und n für 1 oder 0 steht, charakterisiert sind und welche an einer sauerstoff- oder schwefelhaltigen funktionellen Gruppe einer organischen Verbindung gebunden sind, durch die reduktive Auflösung der C-O bzw. C-S-Bindung, dadurch gekennzeichnet, dass man die Reduktion in Anwesenheit einer katalytischen Menge eines Uebergangsmetall-Komplexes einer Corrin- oder Porphin-Verbindung durchführt.

2. Verfahren gemäss Anspruch 1, dadurch gekennzeichnet, dass die Gruppe der Formel I die 2-Bromäthyl-, 2,2-Dichloräthyl- oder 2,2,2-Trichloräthylgruppe ist.

3. Verfahren gemäss Anspruch 1, dadurch gekennzeichnet, dass die Gruppe der Formel I die 2-Chloräthylgruppe ist.

4. Verfahren gemäss einem der Ansprüche 1 bis 3, dadurch gekennzeichnet, dass das Sauerstoff- bzw. Schwefelatom, an welches die abzuspaltende Gruppe der Formel I gebunden ist, das endständige Glied eines Anion-bildenden Rests einer Carbonsäure, Carbaminsäure, phosphorhaltigen Säure, eines Carbonats, Sulfats oder Alkohols bzw. einer entsprechenden Thiocarbonsäure, Dithiocarbonsäure oder eines Thiols ist.

5. Verfahren gemäss einem der Ansprüche 1 bis 4, dadurch gekennzeichnet, dass man als Katalysator Cyanocobalamin, Aquocobalamin oder [1-Hydroxy-2,2,3,3,7,7,8,8,12,12,13,13,17,17,18,18-hexadecamethyl-10,20-diazaoctahydroporphinato]-dibromo-cobalt(III) verwendet.

6. Verfahren gemäss einem der Ansprüche 1 bis 5, dadurch gekennzeichnet, dass man die Abspaltung durch kathodische Elektroreduktion oder durch die Reduktion mit Zink in einem praktisch neutralen Milieu vornimmt.

7. Verfahren gemäss einem der Ansprüche 1 bis 6, dadurch gekennzeichnet, dass man die Gruppe der Formel I als Bestandteil eines Esters zum vorübergehenden Schutz einer Carboxylgruppe verwendet.

8. Verfahren gemäss einem der Ansprüche 1-6, dadurch gekennzeichnet, dass man eine oder zwei Gruppen der Formel I als Bestandteil eines Phosphat-(mono- oder di-)-esters zum vorübergehenden Schutz einer Phosphatgruppe verwendet.

9. Verfahren gemäss einem der Ansprüche 1-6, dadurch gekennzeichnet, dass man die Gruppe der Formel I als Bestandteil eines entsprechenden Aethers oder eines gemischten Carbonsäureesters der Teilformel -C(=O)-O-(I), worin (I) die oben definierte Gruppe der Formel I bedeutet, zum vorübergehenden Schutz einer Hydroxylgruppe verwendet.

10. Verfahren gemäss einem der Ansprüche 1-6, dadurch gekennzeichnet, dass man zwei Gruppen der Formel I als Bestandteil einer Acetal- oder Ketalgruppe oder einen einzigen, den beiden Gruppen entsprechenden zweiwertigen Rest als Bestandteil eines 1,3-Dioxolan- bzw. 1,3-Dioxan-Rings zum vorübergehenden Schutz einer Carbonylgruppe verwendet.

11. Verfahren gemäss einem der Ansprüche 1-6, dadurch gekennzeichnet, dass man die Gruppe der Formel I als Bestandteil eines entsprechenden Carbaminsäureesters zum vorübergehenden Schutz einer primären oder sekundären Aminogruppe verwendet.

Europäisches Patentamt

# EUROPÄISCHER RECHERCHENBERICHT

0037373
Nummer der Anmeldung
EP 81 81 0099

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | JOURNAL OF THE CHEMICAL SOCIETY, Section A, Part 2, Seiten 917-1934 1971 London, G.B. H.A.O. HILL et al.: "The chemistry of vitamin $B_{12}$. Part XV. Catalysis of alkyl halide reduction by vitamin $B_{12a}$: Studies using controlled potential reduction", Seiten 1859-1862<br>* Seite 1861, Spalten 1,2, Absätze "Alkyl iodide reduction" und "Alkylcobalamin reduction" *<br>-- | 1 | C 07 C 51/09<br>59/50<br>63/06<br>C 07 B 7/00<br>C 25 B 3/04<br>87/56<br>85/20<br>C 07 F 9/38<br>C 07 C 149/14<br>41/28<br>29/09<br>C 07 D 319/04<br>C 07 C 103/52<br>317/08 |
| | | | RECHERCHIERTE SACHGEBIETE (Int. Cl.³) |
| | HELVETICA CHIMICA ACTA, Band 61, Fasc. 7, 1978, Nr. 243, Seiten 2560-2578 A. FISCHLI: "Cob(I)alamin als Katalysator. I. Mitteilung. Reduktion von gesättigten Nitrilen in wässeriger Lösung"<br>* Seiten 2561-2565, Absatz "2. Cob(I)alamin als Reduktionskatalysator"; Seiten 2569-2570, Absatz "4. Vergleich mit der Aktivität corrinoidhaltiger Enzyme" *<br>-- | 1,5 | C 07 C 51/00<br>51/09<br>59/50<br>63/06<br>C 07 B 7/00 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| | HELVETICA CHIMICA ACTA, Band 62, Fasc. 3, 1979, Nr. 92, Seiten 882-893 A. FISCHLI: "92. Cob(I)alamin als Katalysator. 4. Mitteilung. Reduktion von α,β-ungesättigten Nitrilen"<br>* Seite 882, Zusammenfassung *<br>--<br>./. | 1 | |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 23-06-1981 | KLAG |

Europäisches Patentamt

**EUROPÄISCHER RECHERCHENBERICHT**

Nummer der Anmeldung 0037373

EP 81 81 0099

-2-

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | HELVETICA CHIMICA ACTA, Band 62, Fasc. 7, 1979, Nr. 242, Seiten 2361-2373<br>A. FISCHLI et al.: "242. Cob(I)alamin als Katalysator, 5. Mitteilung (I). Enantioselektive Reduktion α,β-ungesättigter Carbonylderivate"<br>* Seite 2361, Zusammenfassung *<br>---- | 1 | |
| | | | RECHERCHIERTE SACHGEBIETE (Int. Cl. 3) |

EPA Form 1503.2 06.78